# EUROPEAN PATENT APPLICATION

(11) **EP 4 497 475 A1**
(43) Date of publication of application: **29.01.2025**
(21) Application number: 23823470.2
(22) Date of filing: 16.03.2023
(51) Int. Cl.: A61P 31/04, A61P 17/02, A61L 27/36, A61L 15/40, A61K 35/50, A61M 35/00, A61P 29/00

(54) **CELL FOR TREATING BURN AND BURN TREATMENT METHOD**

(30) Priority: 17.06.2022 JP 2022097967
(71) Applicant: National University Corporation University Of Toyama, Toyama-shi, Toyama 930-8555 (JP); SAKURA SEIKI CO., LTD., Chikuma-shi Nagano 387-0015 (JP)
(72) Inventor: KOBASHI Daisuke, Maebashi-shi, Gunma 371-0837 (JP); YOSHIDA Toshiko, Toyama-shi, Toyama 930-0194 (JP); OKABE Motonori, Toyama-shi, Toyama 930-0194 (JP); ARAI Kenichi, Toyama-shi, Toyama 930-0194 (JP); ARAKAWA Masahiko, Chikuma-shi, Nagano 387-0015 (JP)
(74) Representative: Purdylucey Intellectual Property
(86) International application number: PCT/JP2023/010225
(87) International publication number: WO 2023/243166

(57) **Abstract**

It is an object of the present invention to effectively promote growth of granulation tissue at a dermal defect or a subcutaneous tissue defect caused by a severe burn. To achieve this object, burn treatment cells for use in treatment of burns are composed of placenta-derived cells of an animal and promote healing.

## Description

### Technical Field

The present invention relates to cells that are used during treatment of burns for repairing the periphery of dermal tissue (including subcutaneous tissue) that has been lost, and to a burn treatment method for treating burns.

### Background Art

The severity of a burn is determined by the area and depth. Depth is classified into first to third degrees based on the color tone of the surface. A second-degree burn is a burn that reaches the dermis, and a third-degree burn is a state where all of the skin has been damaged.

When the body has suffered second-degree burns to 30% or more of the body surface area or third-degree burns to 10% or more of the body surface area, intensive care at an emergency center becomes necessary.

In the field of emergency surgery, third-degree burns require surgical treatment because damage affects not just the skin but also underlying tissue, nerves, and blood vessels. Normal wound dressings cannot be applied for reasons such as the increased risk of infection. The area damaged by the burn is removed as soon as possible, and skin grafting is performed after the formation of granulation tissue as a graft bed.

However, if wound bed preparation (that is, favorable granulation), which is indispensable when performing grafting, is insufficient, this will often cause problems such as sepsis and lead to deterioration in the patient's condition and even death due to poor regeneration (that is, difficulty in achieving engraftment of transplanted skin).

According to Non-Patent Literature 1 ("Clinical Practice Guidelines for Management of Burn Care" [Revised 2nd Edition], 2015" published by the Japanese Society for Burn Injuries), reported use of wound dressings on burns relates to second degree burns, and there is no positive evidence for the use of wound dressings on third degree burns. Note that wound dressings used for burns are roughly divided into foam materials, fiber materials, and colloidal materials, which are selectively used as appropriate according to their shape and ability to absorb exudate. These materials are all intended to cause favorable formation of granulation tissue.

Amniotic membrane is a tough biological membrane composed of collagen and elastic fibers, and raw amniotic membrane has been reported as a useful dressing material for trauma and burns (see Non-Patent Literature 2).

However, raw amniotic membrane has not been available immediately when needed and has been complicated to store and handle, which has limited its use in actual clinical practice. On the other hand, dried amniotic membrane (or "hyperdry human amniotic membrane": hereinafter, "HD-AM") manufactured by a specific drying process has also been announced (see Patent Literature 1 and Non-Patent Literature 2). Formation of granulation tissue using HD-AM has also been disclosed (see Patent Literature 2).

### Citation List

### Patent Literature

Patent Literature 1:
   Japanese Laid-open Patent Publication No. 2007-54015
Patent Literature 2:
   Japanese Laid-open Patent Publication No. 2021-020868
Patent Literature 3:
   International Patent Publication No. WO2013-077428

### Non-Patent Literature

Non-Patent Literature 1: "Clinical Practice Guidelines for Management of Burn Care" [Revised 2nd Edition], 2015" published by the Japanese Society for Burn Injuries
Non-Patent Literature 2: Gruss, J. S. & Jirsch, D. W: "Human amniotic membrane: A versatile wound dressing". Can. Med. Assoc. J. 118, 1237-1246 (1978).
Non-Patent Literature 3: Okabe, M. et al. "Hyperdry human amniotic membrane is useful material for tissue engineering: Physical, morphological properties, and safety as the new biological material". J. Biomed. Mater. Res. - Part A 102, 862-870 (2014).

### Summary of Invention

### Technical Problem

As described above, until now, no treatment method for third-degree burns has had an effect of preventing infection and promoting favorable granulation, and at present there is no choice but to rely on the vitality and immunity of the patient.

In the treatment of third-degree burns, it is necessary to grow granulation tissue at an early stage, but there is no effective material to achieve this.

For this reason, forming favorable granulation at an early stage and performing a skin graft at an early stage without causing infection are effective in prolonging the life of a patient with serious burns.

The present inventors created burn model animals with second to third degree burns, and conceived the present invention by histologically, immunochemically, and molecularly confirming that placenta-derived cells are capable of effectively promoting granulation growth at dermal defects and subcutaneous tissue defects in the burn model animals.

### Solution to Problem

Cells for burn treatment according to an aspect of the present invention are used for treatment of a burn and are characterized by being placenta-derived cells of an animal, including a human, and promoting healing.

With this configuration, when using the cells as cells for regenerating dermal defects and/or subcutaneous tissue in a patient with a severe burn in particular, the secretion of physiologically active substances, such as inflammatory cytokines and growth factors, is promoted at the wound site of the severe burn. Such inflammatory cytokines serve to prevent infection due to foreign substances from outside. There are also active effects such as promoting the secretion of IL-6, IFN-γ, IL-10 and COX2 (PGE₂), which promote wound healing of a severe burn. It is possible to perform safe regenerative medicine with few ethical issues and less immune rejection due to allogeneic transplantation.

The cells may be characterized by including amniotic mesenchymal stem cells.

This configuration has high differentiation and proliferation capabilities and promotes wound healing of a severe burn.

The cells may be characterized by being used for tissue regeneration for a burn during burn treatment and promoting granulation formation.

The cells may also be characterized by regenerating tissue for a burn and having antibacterial and anti-inflammatory effects.

The cells may be characterized by being included in a solvent for dripping onto a burn wound.

With this configuration, it is easy to place the placenta-derived cells onto a burn wound.

The cells may be characterized by the solvent having high viscosity.

With this configuration, placenta-derived cells can be prevented from flowing away from the burn wound.

The cells may be characterized by the burn being a second-degree burn and/or a third-degree burn.

With this configuration, the cells are used for regenerating dermal defects and/or subcutaneous tissue in a patient with a severe burn in particular, and promote the secretion of physiologically active substances, such as inflammatory cytokines and growth factors, at the wound site of the severe burn. Such inflammatory cytokines serve to prevent infection due to foreign substances from outside. There are also active effects such as promoting the secretion of IL-6, IFN-γ, IL-10 and COX2 (PGE₂), which promote wound healing of a severe burn.

The cells may be characterized in that the cells are capable of being used alone or in combination with a burn dressing and/or a burn regenerative scaffold.

The cells may be characterized in that the burn dressing and/or the burn regenerative scaffold is dried amniotic membrane obtained by drying raw amniotic membrane that surrounds a fetus of an animal, including a human, and is dried amniotic membrane that has been dried to allow storage in air and so that epithelial cells, basement membrane, and connective tissue that compose the raw amniotic membrane are retained when the dried amniotic membrane is immersed in water or a buffer solution and rehydrated.

With this configuration, dried amniotic membrane can function as a scaffold for cells, induce growth factors and/or cell migration-related chemokines, and locally promote anti-inflammation and tissue regeneration by regulating the differentiation into M2 macrophages.

A burn treatment method according to an aspect of the present invention is characterized by promoting healing by placing placenta-derived cells from an animal, including a human, at a burn wound site.

With this method, when using the cells as cells for regenerating dermal defects and/or subcutaneous tissue in a patient with a severe burn in particular, the secretion of physiologically active substances, such as inflammatory cytokines and growth factors, is promoted at the wound site of the severe burn. Such inflammatory cytokines serve to prevent infection due to foreign substances from outside. There are also active effects such as promoting the secretion of IL-6, IFN-γ, IL-10 and COX2 (PGE₂), which promote wound healing of a severe burn. It is possible to perform safe regenerative medicine with few ethical issues and less immune rejection due to allogeneic transplantation.

The burn treatment method may be characterized by the placenta-derived cells including amniotic mesenchymal stem cells.

This configuration has high differentiation and proliferation capabilities and promotes wound healing of a severe burn.

The burn treatment method may be characterized in that the placenta-derived cells are mixed with a solvent and dripped onto the burn wound.

The burn treatment method may be characterized in that at least 100 of the placenta-derived cells are included per 50µL of the solvent.

The burn treatment method may be characterized in that at least 300,000 of the placenta-derived cells are included per 50µL of the solvent.

The burn treatment method may be characterized in that the solvent has high viscosity.

With this method, the solvent including the placenta-derived cells can be prevented from flowing away from the burn wound.

The burn treatment method may be characterized in that placenta-derived cells of an animal, including a human, are placed on a burn wound and dried amniotic membrane, which has been obtained by drying raw amniotic membrane that surrounds a fetus of an animal, including a human, is placed on top of the placenta-derived cells, the dried amniotic membrane having been dried to allow storage in air and so that epithelial cells, basement membrane, and connective tissue that compose the raw amniotic membrane are retained when the dried amniotic membrane is immersed in water or a buffer solution and rehydrated.

With this method, dried amniotic membrane can function as a scaffold for cells, induce growth factors and/or cell migration-related chemokines, and locally promote anti-inflammation and tissue regeneration by regulating the differentiation into M2 macrophages.

The burn treatment method may be characterized by the burn being a second-degree burn and/or a third-degree burn.

With this method, when using the cells as cells for regenerating dermal defects and/or subcutaneous tissue in a patient with a severe burn in particular, the secretion of physiologically active substances, such as inflammatory cytokines and growth factors, is promoted at the wound site of the severe burn. Such inflammatory cytokines serve to prevent infection due to foreign substances from outside. There are also active effects such as promoting the secretion of IL-6, IFN-γ, IL-10 and COX2 (PGE₂), which promote wound healing of a severe burn.

### Advantageous Effects of Invention

With the cells for burn treatment and burn treatment method according to the present invention, it is possible to promote healing at a burn wound of a severe burn.

### Brief Description of Drawings

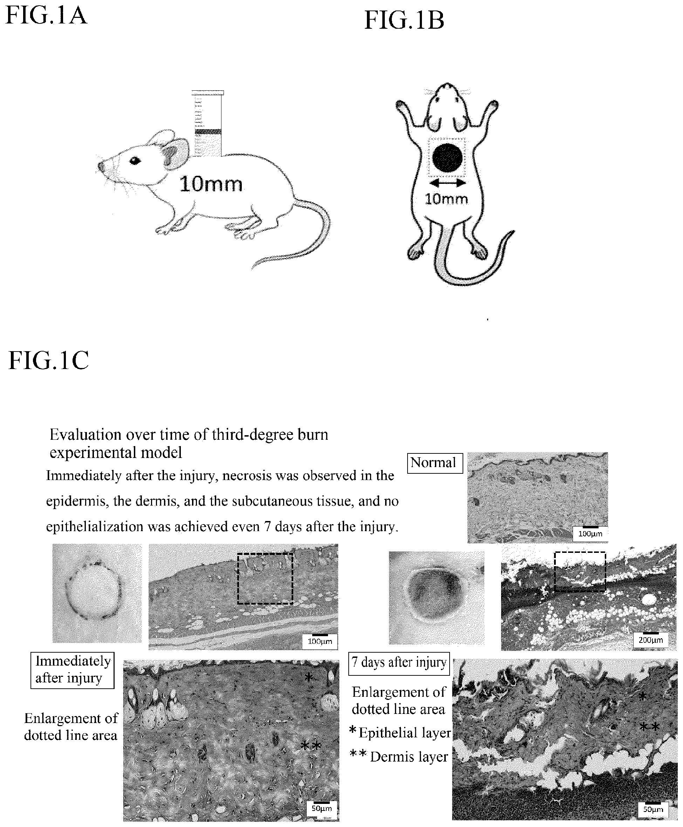

FIG. 1A to 1C are diagrams useful in explaining an experimental mouse model and the application of HD-AM. FIG. 1A and FIG. 1B are diagrams depicting the creation of burn sites on experimental mice. FIG. 1C is a diagram showing the validity of this model as an experimental model for third-degree burns.

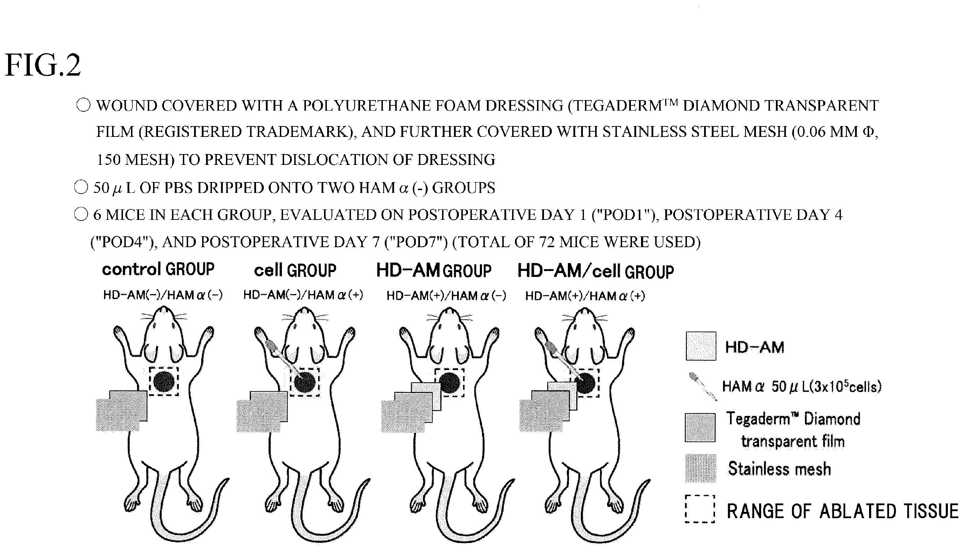

FIG. 2 is a diagram useful in explaining the use of HD-AM and placenta-derived cells (where "Control group" is a schematic diagram of a mouse where HD-AM and placenta-derived cells are not used; "Cell group" is a schematic diagram of a mouse where placenta-derived cells are used; "HD-AM group" is a schematic diagram of a mouse where HD-AM is used; and "HD-AM/Cell group" is a schematic diagram of a mouse where HD-AM and placenta-derived cells are used).

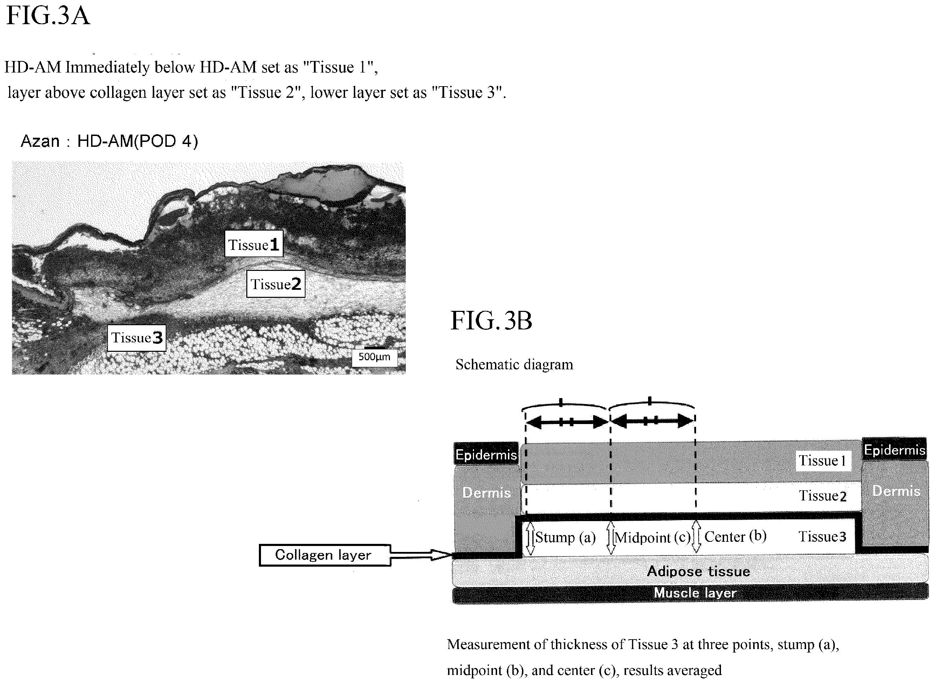

FIG. 3A and FIG. 3B are schematic diagrams of a method for measuring the thickness of granulation tissue. FIG. 3A depicts changes over time in tissue which, following a burn injury, has been stained using the Azan staining method that selectively stains connective tissue. FIG. 3B is a schematic diagram depicting a method for measuring the thickness of granulation tissue formed during treatment after a burn injury.

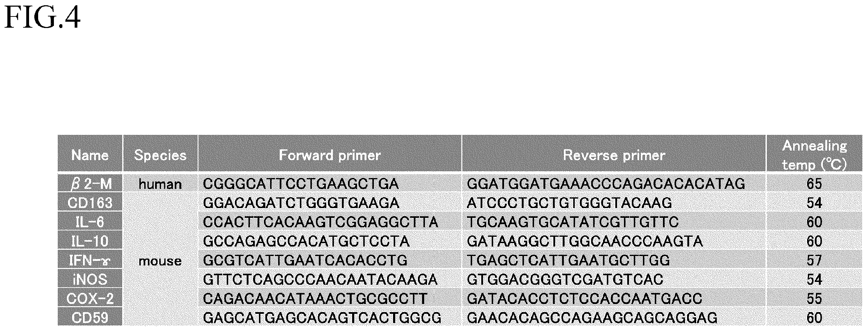

FIG. 4 is a table indicating primers used in quantitative reverse transcription polymerase chain reaction (q RT-PCR).

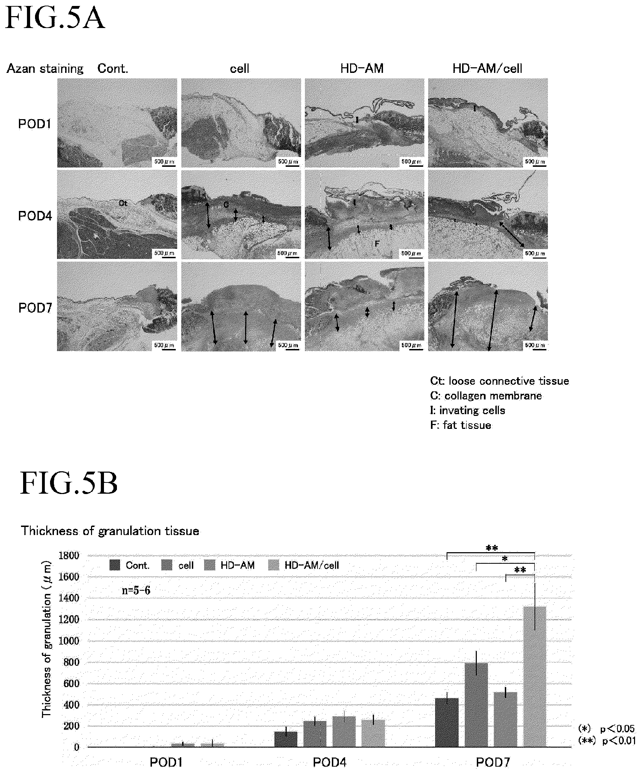

FIGS. 5A and 5B are photomicrographs of granulation formation and a graph comparing mean thickness of granulation tissue over time resulting from treatment following an injury. FIG. 5A is photomicrographs of the Control, Cell, HD-AM, and HD-AM/Cell groups taken 1, 4, and 7 days after treatment (on POD 1, POD 4, and POD 7). The thickness of the granulation tissue is indicated by black arrows. FIG. 5B is a graph comparing the mean thickness values of the granulation tissue.

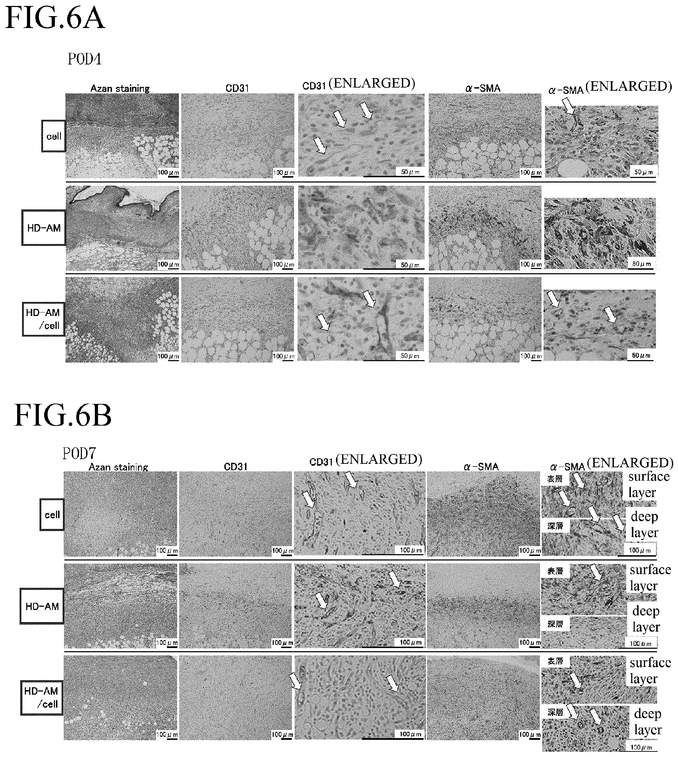

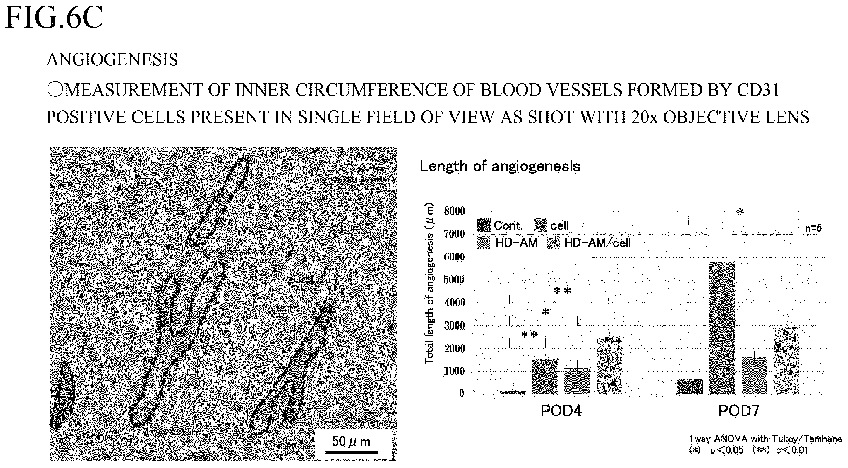

FIG. 6A to FIG. 6C are diagrams useful in explaining angiogenesis in granulation tissue. FIG. 6A is photographs showing the state of angiogenesis on POD4. FIG. 6B is photographs showing the state of angiogenesis on POD7. FIG. 6C is a graph comparing the measurement sites and average measurement values of blood vessels formed in granulation tissue.

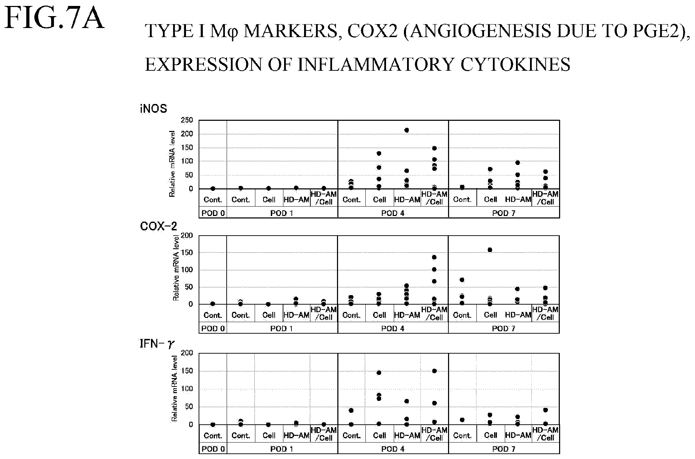

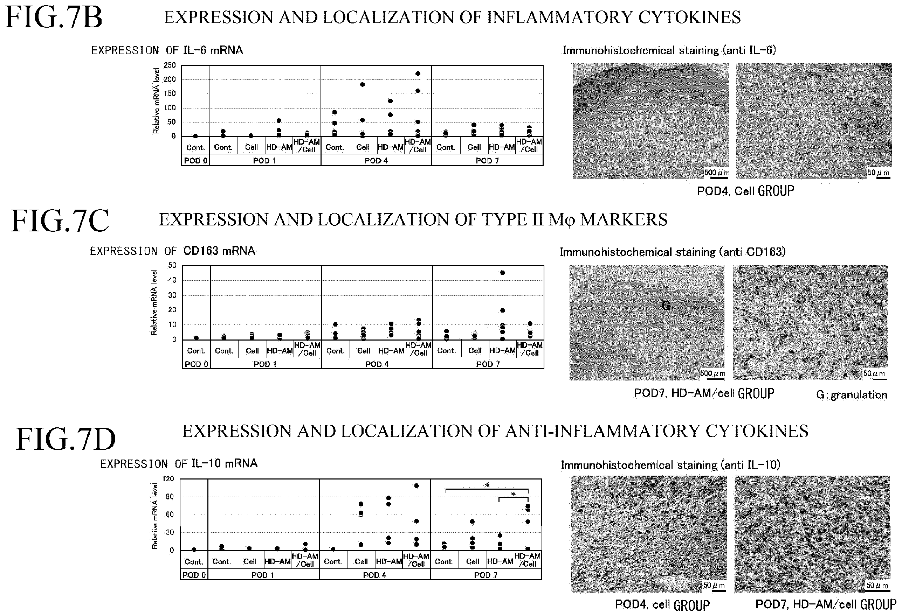

FIG. 7A to 7D are graphs comparing inflammatory and anti-inflammatory cytokines using quantitative RT-PCR. FIG. 7A is graphs depicting mRNA expressions of cytokines involved in inflammation. FIG. 7B is a graph and photographs showing the mRNA expression of the inflammatory cytokine IL-6 and the distribution of IL-6. FIG. 7C is a graph and photographs showing an mRNA expression of a Type II macrophage marker (CD163) involved in anti-inflammation and the distribution of Type II macrophages. FIG. 7D is a graph and photographs showing an mRNA expression of the inflammatory cytokine IL-10 and the distribution of IL-10.

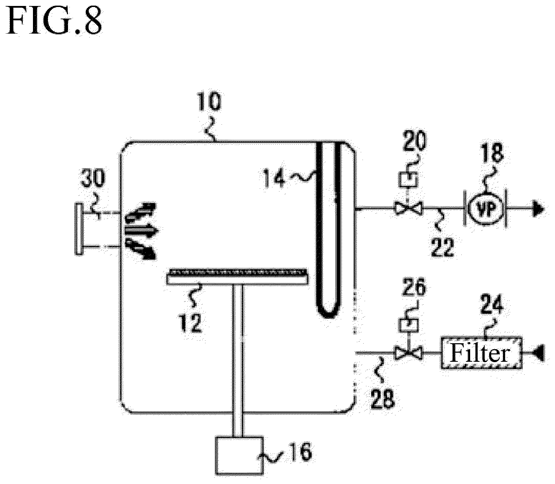

FIG. 8 is a diagram depicting a drying apparatus for producing dried amniotic membrane (HD-AM).

### Description of Embodiments

### Placenta-Derived Cells

First, placenta-derived cells will be described. Although the expression "placenta-derived cells" refers to amniotic mesenchymal cells and amniotic mesenchymal stem cells in the experiments described below, the placenta-derived cells used by the present invention are not limited to such.

Amniotic membrane is an extraembryonic tissue composed of epithelial cells derived from the ectoderm and mesenchymal cells derived from the mesoderm, and contains a group of cells with the properties of pluripotent stem cells. Since amniotic membrane is discarded as waste after birth, there are few ethical issues about using it as a biomaterial. Amniotic membrane has special immunological properties and low immunogenicity, so that the immune rejection reaction following allogeneic transplantation is relatively mild.

Placenta-derived cells are harvested from the amniotic membrane of a mammal, including humans. Amniotic membrane is composed of epithelial cells and mesenchymal cells, and to harvest a population of mesenchymal cells from amniotic membrane, it is sufficient to remove the epithelial cells from the amniotic membrane and perform a separation procedure. As one example, a cell population of mesenchymal cells (MSC) can be collected according to the method described in Japanese Laid-open Patent Publication No. 2003-231639.

Note that as one example, human amniotic membrane can be harvested by Caesarean section from a mother who has given informed consent.

The collected population of amniotic mesenchymal cells contains a mixture of cells with various proliferation capabilities, life spans, and properties. This means that when cellular maintenance is performed on a cell population of mesenchymal cells according to typical culturing conditions, epithelial-like cells that form part of the cell population of mesenchymal cells and will adhere and start to proliferate early in the culture will occupy most of the culture surface. Amniotic mesenchymal stem cells, which begin to proliferate later, are unable to proliferate and are eliminated, which prevents them from being isolated.

For this reason, to prepare a cell population of amniotic mesenchymal stem cells from a cell population of amniotic mesenchymal cells that has been collected, it is possible for example to use the method described in International Patent Publication No. WO2013-077428.

A cell population of amniotic mesenchymal stem cells contains cells with spindle-shaped morphology and a high proliferation capability, and will preferably be capable of 50 or more cell divisions (population doublings).

When placenta-derived cells are used to treat a burn, as one example, it is possible to include 100 or more placenta-derived cells per 50 µL of solvent, or preferably 300,000 placenta-derived cells per 50 µL of solvent and drip the resulting solution onto the burn wound to apply the placenta-derived cells to the burn site.

In addition, when a solution including placenta-derived cells is dripped onto a burn wound, to prevent the solution including placenta-derived cells from flowing away, it is advisable to apply a wound dressing, such as a bandage, to the burn wound after dripping the solution including the placenta-derived cells onto the burn wound. However, since there are cases where normal wound dressings cannot be used on a third-degree burn, dried amniotic membrane (HD-AM), described later, can be used as a dressing to prevent the solution including placenta-derived cells from flowing away. Note that by incorporating placenta-derived cells in a solvent with a high viscosity that still remains fluid, it is possible to prevent the solution including the placenta-derived cells from flowing under gravity away from the burn wound. When placenta-derived cells are incorporated in a high-viscosity solvent, the number of cells that can be incorporated per unit of solvent may be reduced in keeping with the increase in viscosity.

When using placenta-derived cells for the treatment of a burn, the placenta-derived cells may be used alone or in combination with dried amniotic membrane (HD-AM) as described later.

### Dried Amniotic Membrane

One example of dried amniotic membrane that has been manufactured according to a special drying process (called "hyperdrying") is the dried amniotic membrane described in Patent Literature 1. That is, raw amniotic membrane placed in a processing tank is continuously heated by an infrared heater provided inside the processing tank, and a depressurization operation, in which the inside of the processing tank is placed in a depressurized state, and irradiation of the raw amniotic membrane with microwaves from a microwave generating device provided inside the processing tank to apply energy to water molecules present inside the amniotic membrane and cause drying during a pressure recovery operation that slightly raises the pressure inside the depressurized processing tank toward atmospheric pressure were performed. In the dried amniotic membrane (HD-AM) manufactured by repeating the above process a plurality of times, the amniotic membrane cells themselves are inactivated but the cell and tissue structures are retained. In more detail, dried amniotic membrane (HD-AM) is dried so that it can be stored in the atmosphere and so that amniotic membrane produced by rehydrating the dried amniotic membrane by immersion in water or a buffer will retain the structure of epithelial cells, basement membrane, and connective tissue constituting the raw amniotic membrane.

### Combined Use of Placenta-Derived Cells and Dried Amniotic Membrane

Third degree burns in the region of emergency surgery require surgical treatment due to the damage caused not only to the skin but also to the underlying tissue, nerves, and blood vessels. Due to the increased risk of infection, normal wound dressings cannot be used. Areas damaged by burns should be removed as soon as possible, and skin grafting should be performed after granulation tissue has formed to serve as a graft bed.

However, because wound bed preparation (that is, favorable granulation), which is an essential step during transplantation, can be insufficient, it is difficult for the transplanted skin to engraft, which often leads to sepsis and other conditions, causing deterioration of the patient's condition or death.

For this reason, in the present embodiment, experiments were conducted into the formation of favorable granulation tissue using placenta-derived cells alone or in combination with HD-AM.

Placing HD-AM on the wound site of a severe burn as a dressing and scaffolding material was found to protect against infection and promote angiogenesis through the appearance of inflammatory effector cells, including Type I macrophages, and a transient increase in inflammatory cytokines. This then changes to a decrease in the inflammatory cytokines, the appearance of Type-II macrophages, and an increase in anti-inflammatory cytokines, which promotes active effects such as the formation of high-quality granulation tissue.

In the present embodiment, when HD-AM is used as a dressing material and a scaffold, as one example, the HD-AM may be formed into an appropriate shape with scissors or the like in keeping with the wound site of a burn, severe burn, or the like where a normal wound dressing cannot be used. It is advisable to create drainage holes in the applied HD-AM to allow discharge of exudates and the like from the body.

### Creating Burn Models Using Animals

FIG. 1A to FIG. 1C and FIG. 2 are diagrams depicting experimental model mice and the application of HD-AM.

After a mouse has been anesthetized and placed in the prone position, the four limbs are immobilized. Next, the skin on the back was shaved and hair was removed with depilatory cream. Using a tube (see FIG. 1A), a 10 mm area on the back was exposed to hot water at 90°C for 10 seconds to form a third-degree burn wound with a size of ϕ = 10 mm in the center of the back (see FIG. 1B).

FIG. 1C depicts images of an experimental model of third-degree burns, where the exposed skin turned pale immediately after treatment with hot water at 90°C for 10 seconds, and by the seventh day the entire thickness of the skin had necrotized and fallen off. Based on this result, this experimental model was deemed appropriate for use as a third-degree burn model and was used as a third-degree burn model to examine the effects of burn treatment, such as granulation formation.

Note that the handling of laboratory animals was approved by the Institutional Animal Care and Use Committee of the University of Toyama in accordance with the guidelines of the National Institutes of Health. The experiments were conducted according to the guidelines of the Institutional Animal Care and Use Committee of the University of Toyama.

With this method, it is possible to adjust not only the depth of the burn by changing the temperature of the introduced hot water and the exposure time of the hot water, but also the area of the burn by changing the diameter of the tube in use. By doing so, it is possible to easily and stably create burn models as needed, using inexpensive materials for burns that were previously created using expensive equipment.

### Treatment of Burns Using HD-AM and/or Placenta-Derived Cells

FIG. 2 is a diagram useful in explaining the use of HD-AM and/or placenta-derived cells in burn treatment. Note that a case where amniotic mesenchymal stem cells were used as the placenta-derived cells is described below.

In these experiments, four groups were created: a group that used placenta-derived cells (indicated as "Cell group"); a group that used HD-AM (indicated as "HD-AM group"); a group that used HD-AM and placenta-derived cells (indicated as "HD-AM/Cell group"); and a control group that used neither (indicated as "Control group").

Since each group consisted of 6 mice, which were evaluated on postoperative day 1 ("POD1"), postoperative day 4 ("POD4"), and postoperative day 7 ("POD7"), respectively, a total of 72 mice were used.

In the Control group, the burn wound (that is, the wall of exposed bowel) of the mouse model was covered with a polyurethane foam dressing (Tegaderm (registered trademark) Diamond transparent film (registered trademark), made by 3M Deutschland GmbH Health Care Business of Germany) and further covered with a stainless steel mesh (0.06 mm ϕ, 150 mesh).

In the Cell group, 50 µL of a solution containing 300,000 placenta-derived cells was dripped onto the burn wound (that is, the wall of exposed bowel) of the mouse model, this was covered from above with a polyurethane foam dressing (Tegaderm (registered trademark) Diamond transparent film (registered trademark), made by 3M Deutschland GmbH Health Care Business of Germany), and then further covered with a stainless steel mesh (0.06 mm ϕ, 150 mesh).

In the HD-AM group, HD-AM was placed on the burn wound (that is, the wall of exposed bowel) of the mouse model, this was covered from above with a polyurethane foam dressing (Tegaderm (registered trademark) Diamond transparent film (registered trademark), made by 3M Deutschland GmbH Health Care Business of Germany), and then further covered with a stainless steel mesh (0.06 mm ϕ, 150 mesh).

In the HD-AM/Cell group, 50 µL of a solution containing 300,000 placenta-derived cells was dripped onto the burn wound (that is, the wall of exposed bowel) of the mouse model, HD-AM was placed on this and then covered from above with a polyurethane foam dressing (Tegaderm (registered trademark) Diamond transparent film (registered trademark), made by 3M Deutschland GmbH Health Care Business of Germany), and this was then covered with a stainless steel mesh (0.06 mm ϕ, 150 mesh).

Note that as the solvent that includes the placenta-derived cells, it is possible to use saline or phosphate-buffered saline (PBS).

In addition, to prevent the placenta-derived cells from flowing away from the burn wound, it is advisable to include the placenta-derived cells in a cell culturing medium or a gelatinous medium. One example of a gelatinous medium used for culturing cells is agar. The expression "gelatinous" refers to a highly viscous state that has not lost its fluidity. Viscosity may be increased for the saline or phosphate-buffered saline (PBS).

Note that stainless steel mesh was used to cover the HD-AM and or wound dressing material to stop such materials coming off due to the behavior of the mice.

### Histological and Immunohistochemical Staining

To perform histological observation, the burn wound was collected from each mouse on POD 1, POD 4, and POD 7 and embedded in paraffin. Sections sliced from the paraffin block were stained with hematoxylin-eosin (H&E) and Azan. In addition, immunohistochemical staining was also performed for CD31, αSMA, IL-6, CD163, and IL-10. Stained tissue was imaged using a Leica (registered trademark) DMRBE microscope (Leica, Wetzlar, Germany) and a DP73 system (Olympus, Tokyo, Japan).

### Measurement of Granulation Tissue (Regeneration) at the Treatment Location of Burn

FIG. 3A and FIG. 3B are schematic diagrams of a method for measuring the thickness of granulation tissue. FIG. 3A depicts changes over time in tissue which, following a burn injury, has been stained using the Azan staining method that selectively stains connective tissue. The tissue directly below the HD-AM was designated as "Tissue 1", the layer above the collagen layer was designated as "Tissue 2", and the layer below the collagen layer was designated as "Tissue 3".

FIG. 3B is a schematic diagram depicting the measurement position of thickness of granulation tissue that forms at a wound due to treatment after a burn injury.

Note that "Tissue 1", "Tissue 2", and "Tissue 3" are distinguished in FIG. 3A, and the newly constructed granulation tissue in Tissue 3 was measured. The schematic diagram in FIG. 3B shows that Tissue 3 is located on top of adipose tissue.

Azan staining makes it easy to distinguish between collagen fibers and fibrin for observation of newly constructed granulation tissue. The thickness of granulation tissue containing collagen fibers was measured using the Olympus (registered trademark) CellSens (registered trademark) imaging program (version 1.7; Olympus, Tokyo, Japan).

On the 4th day after treatment (POD 4), the appearance of a layer of collagen membrane between the subcutaneous tissue and muscle layer of the surrounding normal tissue was confirmed regardless of whether HD-AM was applied. The regions were determined as depicted in FIG. 3A due to movement of infiltrating cells and granulation formation being observed with this membrane as the boundary.

As depicted in FIG. 3B, changes over time in these regions were observed, measurement was performed at three points a, b, and c where the measurement sites were an epithelium defect stump a, the center b of the epithelium defect, and a midpoint c between a and b. The thickness of granulation tissue formed in the Tissue 3 at the three points a, b, and c was measured, and the mean value was used as the thickness of the formed granulation tissue.

Quantitative Reverse Transcription-Polymerase Chain Reaction (qRT-PCR) FIG. 4 depicts sequences of the primers used in quantitative reverse transcription-polymerase chain reaction (qRT-PCR).

To extract mRNA in the granulation tissue at a wound caused by a burn, target sites were selectively collected from each sample. When sampling was performed, the granulation tissue was dissected in the same manner for all specimens, which were made as anatomically uniform as possible so as not to be affected by differences in sampling sites and regions. Total mRNA was extracted from tissue using Isogen II (Nippon Gene Co. LTD., Tokyo, Japan) according to the manufacturer's instructions. 3 µg of the mRNA was treated with deoxyribonuclease I (DNase I, Sigma-Aldrich, Inc., Tokyo, Japan) for 15 minutes at room temperature. cDNA was synthesized using 500 ng of DNase I-treated RNA using the ReverTra Ace qPCR RT Kit (made by Toyobo Co., Ltd., Osaka, Japan). Gene expression was subjected to real-time RT-PCR analysis using Brilliant SYBR Green QRT-PCR Mix (Stratagene; Agilent Technologies Japan Ltd. Japan) using an Mx3000P quantitative polymerase chain reaction (qPCR) system (Stratagene; Agilent Technologies Japan Ltd, Japan).

On POD 1, POD 4, and POD 7, mRNA was extracted from each of six mice in each group and single measurements were made for inducible nitric oxide synthase (iNOS) produced by Type I macrophages, CD163 (an indicator of type II macrophages), the inflammatory cytokines IL-6 and IFN-γ, and COX-2 (which induces PGE2 involved in angiogenesis).

To establish that the variations in cytokines were postoperative changes, tissue was collected immediately after creating six model mice, and the mRNA expression of each primer was examined immediately after the operation as POD 0. The expression of each mRNA was corrected with GAPDH as an internal control, and relative comparisons were made with the mean values of the Control group on POD 7 as a standard.

### Evaluation of Thickness of Granulation Tissue at Burn Treatment Site

FIGS. 5A and 5B are representative photomicrographs and a graph comparing the measured average thickness of granulation tissue following healing after injury.

FIG. 5A shows representative micrographs of the control, Cell, HD-AM, and HD-AM/Cell groups taken one, four, and seven days after treatment (that is, on POD 1, POD 4, and POD 7). The thickness of the granulation tissue at the wounds in each group is indicated by black arrows.

FIG. 5B is a graph comparing the mean values of the measured granulation tissue.

On POD 1, little granulation tissue was observed in any of the groups.

On POD 4, no difference in the thickness of granulation tissue was observed between the treatment groups.

On POD 7, the HD-AM/Cell group exhibited significantly thicker granulation tissue than the other groups (n=5, *p<0.05, **p<0.01).

### Angiogenesis Inside Formed Granulation Tissue

FIG. 6A to FIG. 6C are photographs illustrating angiogenesis inside the granulation tissue.

FIG. 6A depicts the state of angiogenesis on POD 4 in each group except for the Control group. CD31-positive cells and α-SMA-positive cells were observed within the granulation tissue as a whole.

In the Cell group and HD-AM/Cell group, CD31 positive cells were observed forming a vascular lumen, surrounded by α-SMA positive cells.

In the HD-AM group, CD31 positive cells were present, but no vascular lumen was observed.

FIG. 6B depicts the state of angiogenesis on POD 7 for each group except for the Control group.

In the HD-AM group, CD31-positive cells and α-SMA-positive cells were observed to aggregate and form blood vessels inside the granulation tissue.

In the Cell and HD-AM/Cell groups, the number of such cells was reduced and they were observed running as complete blood vessels. In the Cell and HD-AM/Cell groups, blood vessel formation was observed at deeper layers than in the HD-AM group.

FIG. 6C depicts a comparison of angiogenesis inside the granulation tissue. The photograph on the left depicts an example of granulation tissue that has been magnified with a 20x objective lens, and the inner circumference of blood vessels formed by CD31 positive cells in a single field of view was measured for each group on POD 4 and POD 7. The measurement results are depicted in the graph on the right.

In the graph for POD 4, the length of the blood vessels in the Control group on POD4 was extremely short and very close to zero. However, the length of the blood vessels increased significantly in the Cell group, the HD-AM group, and the HD-AM/Cell group. This length was around 1500 µm in the Cell group, around 1100 µm in the HD-AM group, and around 2500 µm in the HD-AM/Cell group.

On POD 7, the Cell group exhibited a significant increase in the length of the blood vessels, reaching a length of approximately 5,700 µm. In the Cell group and HD-AM/Cell group, the number of blood vessels was small, but blood vessels were observed running over a wide area (from the surface to a deep layer).

### (Comparison of mRNA Expression Patterns Related to Type I Macrophages and Type II Macrophage Markers and mRNA Expression Patterns of Inflammatory Cytokines and Anti-Inflammatory Cytokines During the Healing Process)

FIG. 7A to FIG. 7D depict mRNA expressions of cellular markers and cytokines involved in inflammation.

The expression of iNOS (Type I macrophages) tends to increase in the Cell group, HD-AM group, and HD-AM/cell group on POD4, and a tendency of HD-AM/Cell group > Cell group > HD-AM group was observed. On POD7, the expression of iNOS (Type I macrophages) tended to decrease in all groups.

The COX-2 (PGE2) expression tended to increase in all groups on POD4. On POD7, the expression tended to decrease in all groups except the Control group. In this way, COX2, which is present upstream in the synthesis of PGE2 which causes angiogenesis, increased on POD4 and tended to decrease in all groups on POD7, backing up the immunohistochemical (CD31 and αSMA) results.

The expressions of the inflammatory cytokine IFN-γ tended to be higher in the Cell and HD-AM/Cell groups than in the Control and HD-AM groups on POD4. On POD7, the expression tended to decrease in all groups.

FIG. 7B depicts an mRNA expression of the inflammatory cytokine IL-6 and the distribution of IL-6.

The IL-6 mRNA expression increased on POD4 compared to POD1 in all groups, and tended to decrease on POD7.

The results (on POD 4 for Cell group) of immunohistochemical staining indicated that IL-6-positive cells were diffusely present inside the granulation tissue.

FIG. 7C depicts an expression pattern of the Type II macrophage marker-related mRNA (CD163) and the distribution of CD163-positive cells.

The CD163 (Type II Mϕ) expression was almost nonexistent on POD1, but tended to increase in all groups on POD4. On POD7, the expression tended to increase in the HD-AM group.

Immunohistochemically stained granulation tissue (POD7, HD-AM/cell group) is depicted on the right, which indicates that CD163-positive cells were prominently observed in the newly formed granulation tissue on POD7.

FIG. 7D depicts an mRNA expression of the anti-inflammatory cytokine IL-10 and the distribution of IL-10.

On POD7, the IL-10 mRNA expression was significantly higher in the HD-AM/Cell group compared to the control group and the HD-AM group.

The experimental results described above indicate that for third-degree burns, which are severe burns, placenta-derived cells, either alone or in combination with HD-AM, promoted the formation of granulation tissue that is rich in blood vessels in the subcutaneous tissue.

### Manufacturing of HD-AM

Using the drying device depicted in FIG. 8, raw amniotic membrane was dried to form dried amniotic membrane (HD-AM) with vacuum, far-infrared, and microwave irradiation devices set at the conditions given below.

Raw amniotic membrane that has been placed on a rotating table 12 inside a processing tank 10 is continuously heated by a far infrared heater 14 provided inside the processing tank 10 while performing a depressurization operation that reduces the pressure inside the processing tank 10 and irradiation of the raw amniotic membrane with microwaves from a microwave irradiating device 30 provided inside the processing tank to apply energy to the water molecules present inside the amniotic membrane and dry the amniotic membrane during a pressure recovery operation that slightly raises the pressure inside the depressurized processing tank 10 toward atmospheric pressure. By repeating the above process a plurality of times, it is possible to improve storage stability and handling of the dried amniotic membrane while preserving the cell and tissue structure.

### Drying tank heating temperature: 50°C, FIR: 50°C, Stop valve: 37%, Maximum ultimate pressure 0.34kPa, Dry running maximum ultimate pressure: 0.33kPa

### Drying method

(1) Depressurization 180sec
(2) Pressure recovery 30 sec (with stop valve 37% open)
   Commence emission of microwaves
   0.1kw for 180sec (pressure recovery continues)
(3) Depressurization 180 sec
(4) Repeat (2) and (3) thereafter
(5) End drying manually by checking the ultimate pressure after 180 sec of depressurization in (3) is (0.30 to 0.35 kPa).

The pressure is restored to atmospheric pressure to end the process.

### Industrial Applicability

Placenta-derived cells derived from the placenta of an animal, including humans, are used as a regenerative biomedical material, particularly in the treatment of severe burns. In addition, by using dried amniotic membrane as a medical device in combination with placenta-derived cells, it is possible to cover a dermal defect or subcutaneous tissue of a severely burned patient on which placenta-derived cells have been placed,
and by using this for tissue regeneration (that is, favorable granulation formation accompanied by angiogenesis) during skin grafting, it becomes possible to perform skin grafting, which is essential for third-degree burns or the like, at an earlier stage. In addition, by using amniotic membrane to prevent infection at a burn wound, it is possible to increase the patient's survival rate and achieve therapeutic effects (that is, eliminate the risk of keloids).

## Claims

1. Cells for burn treatment that are used in treatment of a burn and are **characterized by** being placenta-derived cells of an animal, including a human, and promoting healing.

2. The cells for burn treatment according to claim 1,
**characterized in that** the cells include amniotic mesenchymal stem cells.

3. The cells for burn treatment according to claim 1,
**characterized by** being used for tissue regeneration for a burn during burn treatment and promoting granulation formation.

4. The cells for burn treatment according to claim 1,
**characterized by** regenerating tissue for a burn and having antibacterial and anti-inflammatory effects.

5. The cells for burn treatment according to claim 1,
**characterized by** being included in a solvent for dripping onto a burn wound.

6. The cells for burn treatment according to claim 5,
**characterized by** the solvent having high viscosity.

7. The cells for burn treatment according to claim 1,
wherein the burn is a second-degree burn and/or a third-degree burn.

8. The cells for burn treatment according to claim 1,
**characterized in that** the cells are capable of being used alone or in combination with a burn dressing and/or a burn regenerative scaffold.

9. The cells for burn treatment according to claim 8,
**characterized in that** the burn dressing and/or the burn regenerative scaffold is dried amniotic membrane obtained by drying raw amniotic membrane that surrounds a fetus of an animal, including a human, and is dried amniotic membrane that has been dried to allow storage in air and so that epithelial cells, basement membrane, and connective tissue that compose the raw amniotic membrane are retained when the dried amniotic membrane is immersed in water or a buffer solution and rehydrated.

10. A burn treatment method,
**characterized by** promoting healing by placing placenta-derived cells from an animal, including a human, at a burn wound site.

11. The burn treatment method according to claim 10,
**characterized by** the placenta-derived cells including amniotic mesenchymal stem cells.

12. The burn treatment method according to claim 10,
**characterized in that** the placenta-derived cells are mixed with a solvent and dripped onto the burn wound.

13. The burn treatment method according to claim 12,
**characterized in that** at least 100 of the placenta-derived cells are included per 50µL of the solvent.

14. The burn treatment method according to claim 13,
**characterized in that** at least 300,000 of the placenta-derived cells are included per 50µL of the solvent.

15. The burn treatment method according to claim 12,
**characterized in that** the solvent has high viscosity.

16. The burn treatment method according to claim 10,
**characterized in that** placenta-derived cells of an animal, including a human, are placed on a burn wound and dried amniotic membrane, which has been obtained by drying raw amniotic membrane that surrounds a fetus of an animal, including a human, is placed on top of the placenta-derived cells, the dried amniotic membrane having been dried to allow storage in air and so that epithelial cells, basement membrane, and connective tissue that compose the raw amniotic membrane are retained when the dried amniotic membrane is immersed in water or a buffer solution and rehydrated.

17. The burn treatment method according to claim 10,
wherein the burn is a second-degree burn and/or a third-degree burn.
